# EUROPEAN PATENT APPLICATION

(11) **EP 1 712 221 A1**
(43) Date of publication of application: **18.10.2006**
(21) Application number: 05704288.9
(22) Date of filing: 24.01.2005
(51) Int. Cl.: A61K 9/127, A61K 47/10, A61K 47/12, A61K 47/24, A61P 9/10, A61P 7/00

(54) **SUPPORT ACCUMULATING IN INJURED PART IN VASCULAR CHANNEL**

(30) Priority: 23.01.2004 JP 2004015713
(71) Applicant: Keio University, Tokyo 108-8345 (JP)
(72) Inventor: SUEMATSU, Makoto, School of Medicine, Keio Univ., Shinjuku-ku, Tokyo 1608582 (JP); TAKEOKA, Shinji, School of Medicine, Keio Univ., Shinjuku-ku, Tokyo 1608582 (JP)
(74) Representative: Woods, Geoffrey Corlett
(86) International application number: PCT/JP2005/001291
(87) International publication number: WO 2005/070395

(57) **Abstract**

The present invention provides a carrier that accumulates on a damaged tissue and that functions as a drug for controlling a platelet function, a drug delivery method using the carrier and a pharmaceutical composition comprising the carrier. The present invention also provides a carrier with a non-cationic surface, a drug transporter and a pharmaceutical composition comprising the carrier.

## Description

### FIELD OF THE INVENTION

The present invention relates to a non-cationic carrier that attaches to and accumulates on a damaged tissue and fills in the damaged site, to a pharmaceutical composition comprising such carrier incorporating or carrying a drug, and to a drug delivery method.

### BACKGROUND OF THE INVENTION

Thrombus formation is an essential function in an organism in that it closes wound and stops bleeding. Thrombus formation, however, is a main cause of circulatory system diseases due to its properties of narrowing a vascular caliber and interrupting a blood flow. For example, when the outer wall of the blood vessel is damaged, thrombus formation plays a role in a biological defense function for maintaining the perfusion pressure necessary for blood circulation. On the other hand, damage to vascular endothelial cells caused, for example, by peroxidative response causes arteriosclerosis, and significant development of such arteriosclerosis induces thrombus formation. Narrowing of a blood vessel caused by the thrombus and the resultant ischemia could be a main cause of morbidity and mortality. Furthermore, a hemostatic reaction by thrombus formation is deteriorated by thrombocytopenia associated with the use of cancer drugs or blood disorders such as leukemia or by deterioration of blood coagulation factor associated with liver disorder and results in diapedetic hemorrhage from a space between vascular endothelial cells, under such circumstance bleeding in the brain blood vessels or else can lead to lethal change.

A clinically effective use of a drug that prevents blood vessel narrowing requires the drug to reach the damaged vascular endothelium site at a concentration sufficient to inhibit proliferation of smooth muscle cells. However, when this drug is to be systemically administered by intravenous or oral administration, the dose of the drug is limited such that no systemic side effect is produced, in which case a desired outcome may not result. On the other hand, in order to control bleeding tendency, it is necessary to selectively deliver a drug to local damaged vascular endothelial cells where erythrocytes are likely to leak for the same reason.

Conventionally, development has been desired of a drug carrier that allows specific accumulation of a drug at a sufficient concentration on a damaged vascular endothelium site, i.e., a drug carrier having a high degree of specific accumulation property on the damaged vascular endothelium site with a high drug inclusion capacity. For accumulation on a damaged vascular endothelium site, cationization of a liposome surface is known to result in accumulation of the liposome on the damaged vascular endothelium site (Japanese Laid-Open Patent Application No. 7-89874). However, since liposomes with positively charged surfaces coagulate in the blood and distribute as a mass with the platelets, they are difficult to be used as a drug carrier for achieving an effective drug delivery.

Recently, functions of platelets in thrombus formation, leukocyte adhesion, blood coagulation and fibrinolysis have been studied, and *in vitro* elucidations of these processes are making progress. However, the processes found *in vitro* have not been observed in *in vivo* studies since there is no means for direct observation. For this reason, a system was developed that uses a high-speed confocal microscope for imaging thrombus formation in a mouse microcirculation in real time (Falati, S. et al., Nature Med., 8(10), 1175-1180 (2002), Celi, A. et al., J. Thromb. Haemost., 1, 60-68 (2003)). This system allows observation inside the blood vessel in multi-color and in real time, and acquisition of images of a plurality of fluorescent probes and data at the same time via bright field channels. With this system, blood vessel damage can be made with laser under the microscope.
Therefore, *in vivo* studies on platelet aggregation, behavior of tissue factors and fibrins in thrombus formation process and the like are making progress using a vascular endothelium model damaged with laser and a fluorescent and bright field microscope.

### SUMMARY OF THE INVENTION

The objectives of the present invention are to provide a non-cationic surface carrier that accumulates on a damaged tissue and that functions as a drug for controlling platelet functions, to provide a drug delivery method using the carrier and to provide a pharmaceutical composition comprising the carrier.

The present invention also provides a method for directly proving a drug delivery of a so-called drug delivery system in a local area of an organism by observing the behavior of the carrier within the organism in multi-color and in real time using a high-speed confocal microscope.

The present inventors devoted themselves to keen studies to solve the problems mentioned above. As a result, they succeeded in observing behavior of a carrier within a blood vessel in real time and found that the carrier specifically accumulates on a damaged blood vessel site, thereby completing the present invention.

Thus, the present invention is as follows.
(1) A carrier with a non-cationic surface, which can attach to and accumulate on a damaged site of a tissue and/or fills in the damaged site.
   Such carrier may be one with a membrane surface.
   Such tissue may be, for example, a vessel such as a blood vessel.
   Such carrier may be one that can diffuse outside a vessel.
   The damage may reach, for example, the endothelial cells. Examples of such damage include those that result from laser, inflammation (e.g., edema (brain edema, etc.), fever, redness, etc.), ischemic disorder (e.g., cerebral ischemic disorder), ischemia-reperfusion damage (e.g., ischemia-reperfusion-induced organ damage etc.), disseminated intravascular coagulation syndrome, sepsis, bacterial toxin, oxidative stress, tumor or thrombus formation, or bleeding.
(2) A drug transporter comprising the carrier according to (1) above.
(3) A pharmaceutical composition comprising the drug transporter according to (2) above incorporating or carrying a drug.
   The pharmaceutical composition may function as a drug for controlling platelet functions. Examples of such platelet functions to be controlled include hemostasis, antithrombotic action, thrombolysis or antiatherogenic action. Since the carrier according to (1) above is effective in filling in a damaged vascular site, the carrier itself may be used as a drug for hemostasis purpose.
   A drug incorporated in or carried by the carrier may be, for example, at least one selected from a group consisting of: substances that are activated by light such as visible light or ultraviolet light, change in temperature, change in pH, ultrasound, uptake by inflammation-mediating cell or enzyme degradation; hemostatic agents; antithrombotic agents; thrombolytic agents; antitumor agents; and antiatherogenic agents. Examples of the inflammation-mediating cells include lymphocytes, leukocytes, macrophages or platelets.
(4) A drug delivery method comprising allowing the pharmaceutical composition according to (3) above to accumulate on a damaged site of a tissue, or a drug control method comprising allowing the pharmaceutical composition according to (3) above to accumulate on a damaged site of a tissue and allowing the drug to act on the damaged site.
   The action of the above-mentioned drug may be controlled by accumulation of the carrier, diffusion of the carrier or activation of the carrier.
   An example of the above-mentioned tissue includes a vessel such as a blood vessel.
(5) A method for assessing a function of a carrier comprising observing the behavior of the carrier within a tissue under a high-speed confocal widefield microscope.

The above method may comprise observing the carrier behavior within the tissue in multi-color and in real time.

Examples of the above-mentioned carriers include one with a non-cationic surface as well as one with a membrane surface.

An example of the above-mentioned tissue includes a vessel such as a blood vessel.

### BRIEF DESCRIPTION OF THE DRAWINGS

Figure 1 shows the sites for gray level determination.
Figure 2 shows Rh-lipo behavior following ablation of arteriole.
Figure 3 shows Rh-lipo behavior following ablation of venula.
Figure 4 shows diagrams of exemplary analyses of liposome accumulation on damaged sites of arterioles.
Figure 5 is a diagram showing results from an analysis of liposome accumulation on a damaged site of arteriole.

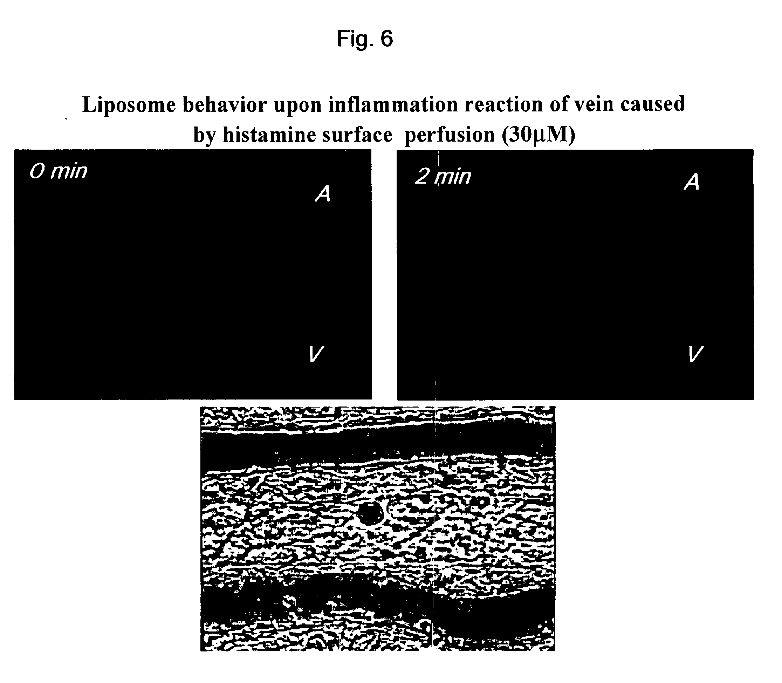

Figure 6 shows liposome behavior at histamine-damaged sites of arterioles and venulae. Upper left panel: following liposome administration and prior to histamine surface perfusion (A: arteriole, V: venula); upper right panel: following liposome administration and 2 minutes after histamine surface perfusion. Large pores open like bamboo joints with respect to venula, and are filled with rhodamine-labeled liposomes; lower panel: leukocyte infiltration occurs following histamine administration but, after liposome administration, leukocytes result in rolling and adhesion and remain within the blood vessel.
Figure 7 is a graph showing relative plasma leakage in brain edema models and other models.
Figure 8 is a graph showing plasma leakage in brain edema models and other models.
Figure 9 is a graph showing plasma leakage in brain edema models.

### DETAILED DESCRIPTION OF THE INVENTION

Hereinafter, the present invention will be described in detail.

### 1. General description

The present invention was completed with a technique using a high-speed confocal microscope for observing, in multi-color and in real time, a model having a thrombus formed by damaging a vascular endothelium by localized laser beam irradiation. This technique allows study of behavior of components involved in thrombus formation such as platelets and behavior of a carrier at a damaged blood vessel site.

Although controlling dynamics of a carrier within a blood vessel has conventionally been considered difficult. New findings on dynamics of a carrier within a blood vessel were acquired by applying the above-described technique to a carrier with a non-cationic surface (preferably the surface is a membrane) (herein referred to as a "non-cationic surface carrier"). Specifically, a non-cationic surface carrier was found to show similar behavior to platelets in that it does not accumulate on a non-damaged site of a blood vessel but specifically accumulate on a damaged site of the blood vessel. It was also revealed that the non-cationic surface carrier shows behavior of accumulating on and filling in a damaged site independently from the platelets for it accumulates while drawing a boundary from the platelets. This allows the non-cationic surface carrier to be used as a drug for controlling a platelet function and as a platelet replacement aiming hemostasis. Furthermore, introduction of a drug into the carrier allows the carrier to be used as a transporter for an antiplatelet agent, an antitumor agent or the like. When a drug that is activated by temperature, pH or the like is introduced into the carrier and a target site of the drug is damaged with laser, the carrier accumulates on the target site, at which point the drug is activated through a change in temperature, pH or the like, thereby delivering the drug more specifically to the target site.

While platelets do not diffuse outside the damaged blood vessel site, the carrier of the invention that accumulated on the damaged site partially diffuses outside the blood vessel. Thus, the carrier of the invention can be a drug transporter that allows a drug to reach outside a damaged vascular site.

### 2. Carrier

According to the present invention, a carrier refers to a liposome, an emulsion, a lipid vesicle or the like. Hereinafter, the present invention is described taking liposomes as an example.

### (1) Preparation of carrier

A non-cationic surface liposome is a liposome with a non-cationic membrane surface, and refers to a stable molecular assembly obtained by dispersing a lipid alone or as a mixture with cholesterol or other amphiphilic molecules in an aqueous vehicle, which (i) has a membrane formed through hydrophobic interaction between hydrophobic parts, and (ii) is highly filled with molecules. Formation of a stable liposome allows, for example, an inner aqueous phase to incorporate a water-soluble drug, or a membrane surface to carry a recognition site. Thus, the liposome of the invention can be utilized as a drug delivery system.

As described above, according to the present invention, the membrane of liposome is non-cationic. In this case, the lipid constituting the membrane of the liposome of the invention may be, as long as the surface of the liposome is not cationic, a phospholipid alone or a mixed lipid of a phospholipid and cholesterol or a fatty acid. Liposome can be prepared by employing a general process such as vortex, ultrasound irradiation, high pressure pumping, high pressure extrusion, forced agitation (homogenizer), freezing-and-thawing method, organic solvent injection, surfactant removal, reversed-phase evaporation, microfluidizer or the like.

A component of a liposome with a non-cationic membrane may be either a saturated phospholipid or an unsaturated phospholipid as long as the surface of the liposome is not cationic (Japanese Patent No. 2936109). Examples include natural phospholipids such as hydrogenated egg-yolk lecithin and hydrogenated soybean lecithin and derivatives thereof, dimyristoylphosphatidylcholine, dipalmitoylphosphatidylcholine, distearoylphosphatidylcholine, dioleoylphosphatidylcholine, dilinoleoyl-phosphatidylcholine, phosphatidic acid, phosphatidylethanolamine, phosphatidylglycerol, phosphatidylinositol may be exemplified. These phospholipids are selected from polymerizable phospholipids having polymerizable groups. Examples of such polymerizable phospholipids include 1,2-di(octadeca-trans-2,trans-4-dienoyl)phosphatidylcholine, 1,2-di(octadeca-2,4-dienoyl)phosphatidic acid and 1,2-bis-eleostearoyl phosphatidylcholine. In this case, the polymerizable phospholipid may have a non-polymerizable long chain such as a linear or branched-chain alkyl group, acyl group, non-polymerizable alkenyl group and non-polymerizable alkenoyl group having a carbon number of 2 to 24. As a fatty acid, a saturated or unsaturated fatty acid with a carbon number of 12 to 20 is used. Examples include myristic acid, palmitic acid, stearic acid, oleic acid, linoleic acid, linolenic acid and octadeca-2,4-dienoic acid.

Moreover, a negatively charged lipid may be used as the lipid constituting the membrane of the liposome, including, for example, diacylphosphatidylglycerol, diacylphosphatidic acid, diacylphosphatidylinositol, diacylphosphatidylserine and fatty acid. In this case, the content of the negatively charged lipid is not particularly limited, but is preferably 1 to 50 mol%, more preferably 5 to 20 mol%.

Furthermore, a stabilizer may be added as an additional component of the membrane-constituting lipid of the liposome. Such stabilizer is preferably sterol and specifically includes ergosterol and cholesterol, preferably cholesterol. The content of cholesterol is not particularly limited, but preferably 10 to 50 mol% for effective stabilization of the liposome membrane.

The stabilizer may be a generally used stabilizer such as dipalmitoyl phosphatidyl glycerol (DPPG) or palmitic acid (PA). The chemical formulae of DPPG and PA are shown below.

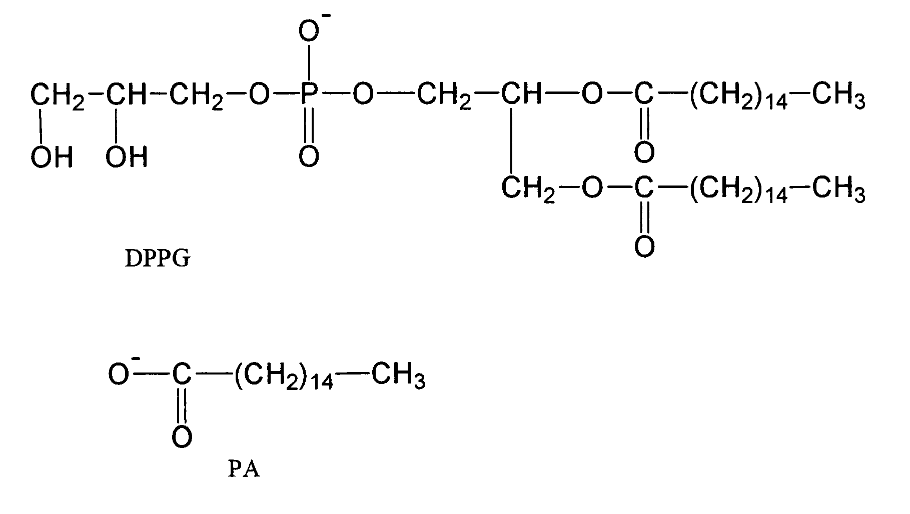

A carboxylic type lipid that has no phosphate group may also be used as the stabilizer. Such stabilizer containing a carboxylic type lipid has at least one of the following features when contained in the liposome: (i) that liposome has no platelet activation action *in vivo,* (ii) that *in vivo* administration of liposome does not reduce the number of platelets in the circulating blood, (iii) that liposome does not induce transient adhesion reaction of platelets *in vivo,* and (iv) that liposome has no leukocyte adhesion activation action *in vivo.* The present invention comprises such stabilizers. An example of the carboxylic type lipid mentioned above preferably includes a lipid expressed by the following general formula (1). 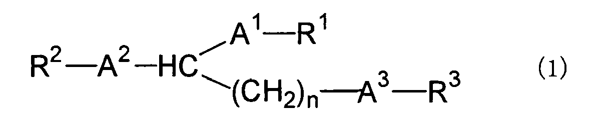 (in formula (1), any one of R¹, R² and R³ is a group expressed by the following general formula (1') 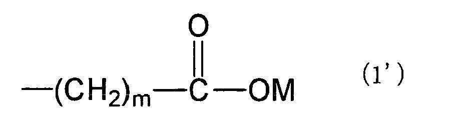 (in formula (1'), M is a hydrogen atom or a monovalent cation, and m is an integer of 1 to 5 indicating the length of the methylene chain), and the other two are hydrocarbon groups (e.g., chain hydrocarbon groups),
A¹, A² and A³ are independently the same or different groups selected from C(O)O, CONH and NHCO, and
n is an integer of 1 to 3 indicating the length of the methylene chain).

The chain hydrocarbon group is not particularly limited and may be, for example, a linear or branched saturated acyclic hydrocarbon group or unsaturated acyclic hydrocarbon group with a carbon number of 2 to 20 (including acyclic terpene), including, for example, various hydrocarbon groups that can be classified into an alkyl group, an alkenyl group, an alkynyl group, an alkadienyl group, an alkylidene group, an alkylidine group or the like.

In general formula (1), binding sites of R¹, R² and R³ are preferably trifunctional amino acids such as lysine, asparagine, glutamine, aspartic acid, glutamic acid, serine, threonine and tyrosine. Especially, a trifunctional amino acid having one reactive functional group and two equally reactive functional groups is particularly preferable, specifically, aspartic acid or glutamic acid having one terminal amino group and two terminal carboxyl groups, or lysine, asparagine and glutamine having one terminal carboxyl group and two terminal amino groups. Aspartic acid and glutamic acid are particularly preferable, and homocysteine and glutathione may also be used.

An example of the carboxylic type lipid represented by general formula (1) preferably includes DPEA (1,5-dipalmitoyl-L-glutamate-N-succinic acid (also referred to as DHSG (1,5-o-dihexadecyl-N-succinyl-L-glutamate))) represented by the formula below.

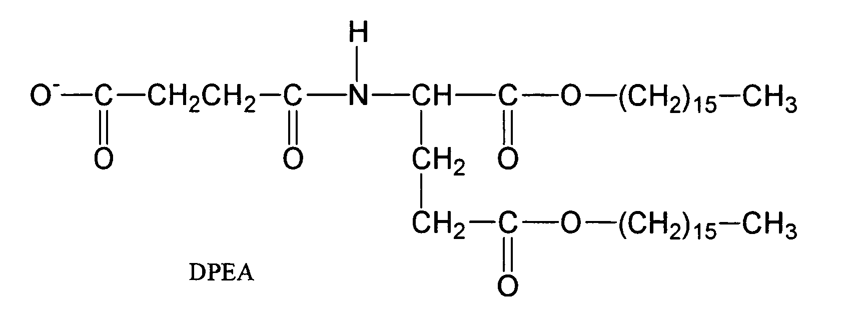

Examples of aqueous vehicles include pure water, an aqueous solution and a buffer solution. The aqueous vehicle may suitably be selected according to the liposome application, but an injectable solution and saline are preferable in terms of safety in an organism. The pH of the aqueous vehicle is not limited as long as it is within a physiological pH range and it does not affect deterioration of stability and change in the aggregate morphology. The range of physiological pH refers to a pH range in an organism, blood or the like, and is, for example, pH 4 to 11, preferably pH 6 to 9.

The liposome of the invention has a particle size of 0.02 to 250 µm, particularly preferably a size of 0.1 to 1.0 µm. For example, the particle size of the liposome may be controlled by adding a water-soluble substance and an aqueous vehicle to mixed lipid powder for hydration and swelling and subsequent stand-alone hydration, a voltex mixer, a forced agitator, an ultrasound irradiator, a homogenizer, a microfluidizer, a high pressure extruder or freezing-and-thawing. In particular, a combination of freezing-and-thawing with a high-pressure extruder significantly enhances the filter permeability, and therefore shortens the processing time and improves the yield. A water-soluble substance that was not incorporated may be separated from the incorporated liposome by gel filtration, ultracentrifugation or a limiting membrane treatment. Examples of the water-soluble substances used with the present invention include water-soluble proteins such as hemoglobin and albumin, peptides, nucleic acids and water-soluble drugs.

### (2) Surface modification

According to the present invention, a suitable modifier can be added to the above-described liposome membrane to modify the membrane. Examples of substances that modify the membrane include fatty acids and fats binding to sialic acid or polysaccharide, and phospholipids, fatty acids and cholesterols binding to polyoxyethylene. Preferably, the membrane is modified with polyoxyethylene (polyethyleneglycol). Surface modification with polyethyleneglycol has been confirmed to improve dynamics of blood flow (Sakai et al., Bioconjugate Chemistry, Vo.8, 23-20, 1997). Polyethyleneglycol chain is highly effective in suppressing the particle size change of liposome in the subsequent step of dispersing dried liposome in an aqueous solution and also in suppressing aggregation. Therefore, polyethyleneglycol is preferable in that it can prevent deterioration of filter permeability and clogging caused by liposome aggregation in the final extrusion step. The content of polyethyleneglycol lipid is preferably 0.01 to 1 mol% of the mixed lipid, more preferably 0.1 to 0.3 mol%. The molecular weight of the polyethyleneglycol is 2,000 to 12,000, preferably about 4,000 to 10,000.

### 3. Tissue damage

### (1) Damage

According to the present invention, the cause of tissue damage is not particularly limited and both extrinsic and intrinsic damage are contemplated. The damage comprises any damage that does harm to the endothelial cells where such damaged site becomes inflamed. Such inflammations include those that are caused by physical and chemical stimulations. Physical stimulations include laser, radiation, cut or the like while chemical stimulations include histamine, leukotriene, thrombin, kinin, active oxygen, oxidative stress, heavy metal, bacterial toxin, cytokine or the like. These inflammations also include edema (e.g., brain edema), fever, pain and redness. Examples of extrinsic damage include blood vessel damage by injury, and damage by laser used for the purpose of disease prevention or treatment. Examples of intrinsic damage include ischemic disorders (e.g., cerebral ischemic disorder), ischemia-reperfusion damages (e.g., ischemia-reperfusion-induced organ damage), disseminated intravascular coagulation syndrome, sepsis, tumors and thrombus formations. In addition, intrinsic damage also includes damage of endothelial cells resulting from peroxidative response caused by various bad factors in blood vessels induced by arteriosclerosis risk factors such as hypertension, hyperlipemia, obesity, diabetes and smoking, as well as the resulting arteriosclerosis lesion.

### (2) Accumulation on tissues

A tissue for accumulating the carrier of the invention is not particularly limited as long as it comprises endothelial cells. A vessel with endothelial cells has a luminal structure with a single endothelial cell layer, and examples of such tissues include blood vessels and lymph vessels, preferably blood vessels since a carrier can readily be infused into blood. The blood vessel may be any one of artery, vein and capillary blood vessel. Hereinafter, a blood vessel is exemplified for the sake of convenience.

The carrier of the invention does not accumulate on an undamaged blood vessel and instead specifically accumulates on an endothelial cell site damaged as described above. The accumulation behavior of the carrier on the damaged part is almost the same as the platelet dynamics, in which it accumulates in a single or multiple layers to cover the damaged part, and penetrates into a subendothelial space from the damaged vascular endothelial cell site for site-specific filling. On the other hand, the carrier of the invention does not interact with the platelets flowing in the blood by nature at the accumulation site and defines a boundary from the region where platelets accumulate, for independent accumulation.

### (3) Partial diffusion

When the endothelial cell damage is associated with damage in the basal membrane of the blood vessel, the carrier not only accumulates on the damaged site inside the blood vessel but a part of it diffuses outside the blood vessel from the damaged site. Therefore, when the degree of the damage is large, the carrier incorporating or carrying a drug not only stays within the blood vessel wall but also diffuses and reaches outside the blood vessel. As a result, the drug can be delivered efficiently to the damaged site, which is favorable for a higher treatment effect. Even when the carrier is not incorporating a drug, it can block the penetrated site of the blood vessel wall from outside the blood vessel and thus a high hemostatic effect may be expected.

### 4.Drug delivery

### (1) Drug

According to the present invention, a drug is incorporated into or carried by a carrier to be used as a pharmaceutical composition. The drug may be a diagnostic substance or a pharmacologically active substance according to the diagnosing and/or treating purpose for the damaged blood vessel site.

According to the present invention, the drug incorporated into or carried by the carrier is not particularly limited as long as the drug does not interfere with the carrier morphology. Specifically, examples of the drugs include hemostatic agents, blood coagulation promoters, antithrombotic agents, thrombolytic agents, antitumor agents, anticoagulant agents, protease inhibitors, prostaglandin formulations, inhibitors of blood vessel smooth muscle cell proliferation, antibiotics, anti-inflammatory agents, antiatherogenic agents, lipid uptake inhibitors, inhibitors of vascular endothelial cell proliferation, vitamins (e.g., vitamins C, E and K), anti-infectives, dermatologic drugs, drugs for sense organs, drugs for endocrine systems, drugs for digestive organs, drugs for circulatory organs, drugs for excretory organs, genital drugs, respiratory drugs, nervous system drugs, diagnostic adjuvants, nutritional supplements, amino acids and proteins, each of which may be used alone or in combination. Target diseases are those that meet the applications of the above drugs. The carrier may also incorporate hemoglobin for artificial erythrocytes or various medicines such as chemotherapy agents for cancer, or the carrier may incorporate a gene (nucleic acids (DNA, RNA)) for the purpose of gene therapy.

In addition, the carrier may incorporate or carry, for example, a drug that is activated by light such as visible light or ultraviolet light or ultrasound, or a drug that is activated by change in temperature or pH. Moreover, the carrier may incorporate or carry a drug that is activated by uptake by inflammation-mediating cells or enzymatic degradation. Examples of inflammations include edema and leukocyte infiltrations (wheal, angioedema, brain edema, brain ischemia). Examples of the above-described inflammation-mediating cells include lymphocytes, leukocytes, macrophages and platelets.

The phrase "a drug is incorporated into the carrier" means that the drug is incorporated into an aqueous vehicle inside the carrier.

When the carrier is a liposome, a drug may be incorporated into the carrier by mixing the drug with the raw lipid in the aqueous vehicle upon carrier preparation as described above.

The phrase "a drug is carried by the carrier" means that a drug is physically attached to or chemically bound to the surface of the carrier. The phrase specifically means that a drug is bound to an amphiphilic site of the membrane itself, such that the drug is stuck into a lipid bilayer membrane with or without penetrating through the membrane, or that the drug is sandwiched (bound) between hydrophobic parts of the lipid bilayer membrane.

The carrier may carry a drug such that the drug is enclosed by a liposome lumen, or the drug is retained inside a lipid double membrane or on a surface layer.

A drug that is activated by light such as visible light and ultraviolet light, change in temperature or pH, ultrasound, uptake by an inflammation-mediating cell (lymphocyte, leukocyte, macrophage or platelet) or enzymatic degradation may be incorporated into or carried by the carrier. This drug activation means increased onset of pharmaceutical activity, drug liberation from the carrier and/or activation of an inactive drug. When a site to be diagnosed or treated is irradiated with laser for locally damaging a blood vessel, the carrier accumulates on that damaged site. When the endothelial cell damage reaches the basal membrane of the blood vessel, part of the carriers diffuse and accumulate outside the blood vessel. A drug incorporated into or carried by the carrier accumulating on the damaged blood vessel site can be activated with light such as visible light and ultraviolet light, change in temperature or pH, ultrasound, uptake by an inflammation-mediating cell (lymphocyte, leukocyte, macrophage or platelet) or enzymatic degradation for effective release of a diagnostic substance or a pharmacologically active substance.

The amount of a drug incorporated into or carried by the carrier of the invention may suitably be determined according to the purpose of the treatment by those skilled in the art. The dose of the pharmaceutical composition of the invention varies and may suitably be selected according to age, sex, condition, administration route, and number of doses. For example, in the case of an antitumor agent, 1 to 5 mg of it can be incorporated into a 1 mL liposome suspension (diameter: 100-300 nm).

The administration route of the pharmaceutical composition of the invention may be a systemic administration such as generally-employed intravenous and intraarterial administrations as well as a local administration such as intramuscular, subcutaneous and intracutaneous administrations. Administration routes using catheters are also possible.

### (2) Function control

When a vascular endothelial cell is damaged, platelets accumulate on the damaged site to form a thrombus for hemostasis. In the early stage, a substance responsible for plasma colloidal osmotic pressure such as albumin leaks from a gap caused between the endothelial cells by vascular endothelium damage and causes edema, and this gap will eventually be an entrance for the inflammation-mediating cells to enter the tissue. Through these reactions, so-called inflammation reaction is induced, resulting in edema, fever, pain or redness. These reactions are known to be caused commonly in a blood vascular system associated with multiple organ failure resulting from cancer dissemination in the blood vessel or sepsis (Katayama T, Ikeda Y, Handa M, Tamatani T, Sakamoto S, Ito M, Ishimura Y, Suematsu M. Immunoneutralization of glycoprotein Ibα attenuates endotoxin-induced interactions of platelets and leukocytes with rat venular endothelium in vivo. Circulation Research 2000, 86, 1031-1037.). In other words, platelets and leukocytes have properties of recognizing and accumulating on a damaged endothelial cell site.

At a site damaged by an extrinsic factor such as laser, platelets accumulate to fill in the blood vessel wall to stop the bleeding, thereby keeping the closed nature of the circulatory system. When the vascular endothelial cell is detached or when the vascular endothelial cell is damaged due to peroxidative response, platelets accumulate and form a thrombus at the damaged site. Platelets have a nature of adhering to a blood vessel site damaged by a tumor, and activating or releasing a substance inside the platelets to form a thrombus at that site. At the damaged site of the vascular endothelium described above, inflammation reaction is induced by leukocytes. Briefly, not only platelets but also leukocytes accumulate on the damaged vascular endothelium site.

Hereinafter, an example of controlling blood vessel function by applying the carrier of the invention that recognizes and accumulates on a damaged endothelial site will be described below relative to physiologic functions of various cells.

### (2-1) Platelets

A carrier of the invention that shows the same adhesion behavior as that of platelets may act as a drug for controlling platelet functions. Controlling a function means to suppress or promote functions of platelets by using the carrier incorporating or carrying a drug appropriate for the diagnosis or treatment purpose in place of platelets. Specifically, the platelet functions to be controlled are hemostasis, antithrombotic formation, thrombolysis, antiatherogenic action and the like.

For example, a carrier that accumulates on an extrinsically damaged site by injury closes the damaged site and exhibits a hemostatic effect. If the carrier incorporates or carries a drug having a hemostatic effect or a blood coagulation promoting effect, the carrier can exhibit a higher hemostatic effect. Thus, the carrier of the invention functions to enhance the hemostatic function of the platelets.

A carrier that accumulates on an intrinsically damaged site can prevent or lyse a thrombus derived from accumulated platelets through the action of the drug incorporated into or carried by the carrier. Thus, the carrier of the invention can function to suppress the accumulation function of the platelets.

### (2-2) Leukocytes

As described above, not only platelets but also leukocytes accumulate on a damaged vascular endothelium site. Since the carrier of the invention shows similar accumulation on a damaged vascular endothelium site to platelets, the carrier is expected to show similar adhesion behavior to that of leukocytes and thus can function as a drug for controlling leukocyte functions. To control leukocyte functions means that a carrier incorporating or carrying a drug that suppresses or promotes physiologic actions of leukocytes functions as a leukocyte. There are mainly three types of leukocytes, namely, lymphocytes, monocytes and granulocytes, which play main roles in immune function. Such leukocyte functions to be controlled include microbial phagocytosis/sterilization/digestion, body's defenses, defense against infection, inflammation action, parasite destruction, promotion of immediate allergic reaction and else.

For example, a carrier that accumulates on an extrinsically damaged site has actions of preventing foreign viruses, microbial invasion and infection. A carrier incorporating or carrying a drug having an antimicrobial action, antibacterial action or the like is capable of exhibiting a higher degree of leukocyte-like function. The carrier can also incorporate various cytokines (e.g., IL-1, IL-2 or IL-4) to activate monocytes or lymphocytes. A carrier that accumulates on an intrinsically damaged site has an action of preventing leukocyte infiltration and a body fluid leakage from a blood vessel to a tissue. The infiltration of leukocytes from a blood vessel is known to cause inflammation of the tissue via cytokine liberation, and the leakage of body fluid is known to cause systemic or local edemas (e.g., leg edema or brain edema). The carrier of the invention exhibits a function of preventing such leukocyte infiltration or leakage of body fluid that can be a cause of inflammation.

Thus, the carrier of the invention also has a function of controlling leukocyte functions.

### (3) Drug action

Since a carrier of the invention partially diffuses outside a blood vessel, it can be effective from outside the external wall of the blood vessel for the treatment of thrombus or damaged blood vessel site.

By adding a fluorescent substance that can be excited by laser to the drug, a site to be diagnosed or treated can readily be identified in laser diagnosis or treatment, thereby facilitating site-specific drug activation. The laser in this case is used for different application from the laser for making damage to a blood vessel.

When the carrier of the invention incorporates or carries a drug that is activated by light such as visible light or ultraviolet light, temperature, pH or the like, the effect of the drug can be exerted at one's selections of desired period, desired site of action and desired action level.

A drug is delivered by being incorporated into or carried by a carrier that accumulates on a damaged blood vessel site so that it can act specifically on the damaged site. The activity of the drug can be controlled by accumulation, diffusion or activation of the carrier. The present invention provides such drug delivery method and drug control method.

### 5. Observation inside a blood vessel using a high-speed confocal widefield microscope

The present invention provides a method for observing behavior of a carrier in an organism under a high-speed confocal widefield microscope. This method allows observation of carrier behavior within a tissue (blood vessel) in multi-color and in real time. In addition to the carrier, platelets or leukocytes (hereinafter, also referred to as "platelets and others") from an organism or a target site may also be fluorescently labeled for simultaneous observations of their behavior. Thus, the method of the present invention is highly effective in studying selectivity, aggregation and dynamics of a carrier within an organism.

### (1) Preparation of subject animals to be observed

Subject animals are not limited as long as their blood vessels can be observed under a confocal widefield microscope, but preferably an animal that is used widely in the studies of drug carrier dynamics. Preferable animals include rat, guinea pig, mouse, hamster, suncus, rabbit, dog, pig, cat and monkey. More preferably, animals are rat, guinea pig, mouse and hamster.

Anesthesia of the subject animals is performed by a method used in general animal experiments. For example, pentobarbital sodium, ketamine, chloral hydrate, urethane, diethyl ether or the like, or a combination thereof may be used. Preferably, an anesthesia method does not exhibit an action on the blood vessel like vasodilating action. Administration into the subject animals may be carried out in use and dose appropriate for each of the sedatives and anesthetics.

A catheter is inserted into the vein of a subject animal under anesthesia, and a compound for fluorescently labeling platelets and others is intravenously administered via the catheter. The insert position of the catheter is not particularly limited but preferably femoral vein in view of catheter stability, subject animal safety and the chance of damage on the observed site.

### (2) Labeling of platelets and others

In order to observe platelet behavior at a damaged blood vessel site with a microscope, the platelets and others are labeled with a fluorescent dye. Examples of compounds for labeling the platelets and others include those that have ester bond in the molecule, for example, carboxylfluorescein diacetate succinimidyl ester: CFDA-SE (CFSE).

The labeled compound is incorporated into the cell for having membrane permeability but does not produce fluorescence at this stage. When CFDA-SE is incorporated into the platelets and others, ester bond is broken by esterase in the cell, by which the compound becomes a fluorophore, thereby emitting strong fluorescence. Once the compound is incorporated into the cell, it stays within the cell and thus cell labels are not eliminated during a long period of assay.

### (3) Labeling of carrier

A fluorescent label agent for the carrier is not particularly limited as long as it presents different color from the compound used for fluorescently labeling the platelets and others above, and includes rhodamine, Texas Red and fluorescein. If the platelets are labeled with CFDA-SE that develops green color, the label for the carrier is preferably rhodamine that develops red color. The carrier to be infused, as a solution containing 35% by volume of the carrier (liposome), is 10 to 2000 µL, preferably 50 to 400 µL and more preferably 250 µL per rat weighing 100 g.

The method for administering the carrier may vary according to purpose, and includes intravenous administration as well as intraperitoneal administration, intramuscular administration, subcutaneous administration, nasal administration, transpulmonary administration, oral administration and topical administration. Furthermore, according to the administration method, a carrier containing a pharmacologically accepted excipient or base may be administered.

### (4) Observation of blood vessel

A fluorescently labeled compound is infused via the above-described catheter. Following the subsequent abdominal surgery, the blood vessel site to be observed is placed on a glass coverslip for *in vivo* observation. The site to be observed is not particularly limited although an ileocecal region of mesenterium is preferable, and also includes circulatory organs such as brain, liver and digestive tract.

The site to be observed is perfused with a buffer generally used for biologic sample (e.g., Krebs buffer). Preferably, the buffer used is saturated with 95% N₂/5% CO₂. The perfusion is carried out under conditions that keep the temperature warm at 37°C so as not to produce effects on the sample.

### (5) Damage on vascular endothelial cells with laser

Next, the fluorescently labeled carrier is infused via the above-described catheter. Within 1 hour, a blood vessel to be damaged is selected. Examples of the blood vessels subjected to damage include an artery, a vein, a capillary blood vessel and a lymph vessel. A laser beam used for making damage is pulsed light of nitrogen-dye laser. For example, laser irradiation that gives light flux with a diameter of 1 µm on the target site under a microscope objective lens gives damage to the vascular endothelial cells in a site-specific manner.

### (6) In vivo imagings of thrombus formation and carrier dynamics

A system required for imaging the damaged blood vessel site may be obtained by modifying the method of Falati et al. (Falati S. et al., Nature Medicine, 8(10), 1175-1180(2002)). This modified system has an improved epi-illumination microscope with a trinocular tube. In order to carry out a test with an *in vivo* microscope, a water immersion lens or an oil immersion lens with a certain numerical aperture is required. Specifically, 60x water immersion lens (LUMFL) with a numerical aperture of 1.1 is preferable.

According to the present invention, when a Nipkow disk technique scanner is used as a confocal microscope, the microscope will theoretically be able to display up to 720 frames per second. An example of the laser for exciting fluorescence includes a dual-line argon/krypton laser. With this laser, fluorescence can be obtained with a confocal microscope having excitation wavelengths at 488nm and 568nm.

When a thrombus is formed with laser, nitrogen dye laser is used. A laser beam is introduced into the light path of epi-fluorescence (epi-illumination port) so that the laser beam is radiated to the endothelial cells of the small blood vessel of the mesenterium via the objective lens. The laser generates 4-nsec energy pulse at a frequency of 3 to 10Hz on the surface of he blood vessel having a diameter of about 0.5 µm. The pulse energy is quantitatively controlled appropriately with a digital controller.

A color CCD camera is connected to the center of the substrate of the trinocular microscope so that a color image can be recorded with high spatial and temporal resolution.

### (7) Image analysis

The fluorescent images are collected by three different ways using exciting light at the excitation wavelength of the platelet-labeling dye, at the excitation wavelength of the carrier-labeling dye and at both excitation wavelengths to obtain images of behavior of the platelets only, behavior of the carrier only and behavior of both of the platelets and the carrier, respectively. The exciting light is a laser beam obtained via a filter set for each dye.

The fluorescent image is first processed with an 8-bit digitizer connected to a computer, and then the pixel-based data is converted to gray level. The gray level of the site of interest is numerically converted with digital imaging software.

Spatial distribution of the fluorescently labeled carrier in the periphery of the laser-damaged blood vessel is assessed by gray level determination. Specifically, line scanning is carried out along the longitudinal axis of the blood vessel and along the cross-sectional direction perpendicular thereto with respect to the damaged site of the blood vessel wall. The line scanning gives the amount of the carriers that leaked out of the blood vessel and the spatial characteristic of the distribution thereof.

Hereinafter, the present invention will be described more specifically by way of examples. The invention, however, is not limited to these examples.

### EXAMPLE 1: Preparation of liposome

One g of mixed lipid powder (DPPC (dipalmitoylphosphatidylcholine)/cholesterol/DPEA/PEG-DSP (N-(monomethoxypolyethyleneglycol-carbamyl)distearoylphosphatidyl ethanolamine) = 5/5/10/0.033 (molar ratio)) was dispersed in 25mL of saline and agitated at room temperature for 10 hours. The resultant was passed through a filter with a pore diameter of 0.22 µm twice in an extruder to obtain liposome dispersion with an average particle size of 263 ± 43 nm. Lipid concentration in saline was 3g/dL. To 20 ml of this dispersion, separately prepared Octadecyl Rhodamine B (from Molecular Probe) in ethanol solution (1mg/1mL) was dropped for 600 µL, while agitating, and further subjected to 2 hours of agitation with light being shielded. Liposomes were precipitated by ultracentrifugation (100,000 g, 60 min. + 150,000 g, 30 min.). A slight amount of unintroduced dye and ethanol remaining in the supernatant were removed. Saline was added to redisperse the vesicles and the resultant was passed through a filter (pore diameter 0.45 µm).

The charge of the membrane of the obtained liposome was measured and zeta potential was found -2.6V

### EXAMPLE 2: Analysis of liposome behavior by image analysis method

### 1. Biomicroscopic system and microcirculation observing/recording system

Rats were bred under the control of the Laboratory Animal Center, School of Medicine, Keio University. According to the method of Suematsu et al. (Suematsu M, et al. Lab Invest 1994), male Wistar rats (200-250g; Clea Japan, Tokyo) were anesthetized by intramuscular injection of pentobarbital sodium at 50 mg/kg, and a catheter was inserted into a femoral vein to administer carboxyfluorescein diacetate succinimidyl ester (CFDA-SE) at 1mg/kg for biostaining of the platelets in the body.

The mesenterium at the ileocecal region was opened extraperitoneally to observe the microcirculatory system with an erecting-type confocal laser microscope.

A 60x objective lens was used. Within the light path of the microscope are implemented an argon laser capable of laser output at 488 nm that allows imaging of the CFSE fluorescence and laser output at 568 nm that allows imaging of Rhodamine B, as well as filters that allow the former green fluorescence alone, the latter red fluorescence alone or both at the same time for imaging the merge, with unrestricted filter switching. An image obtained with transmitted light without the filter was also possible. In addition, a nitrogen dye laser whose output can be freely and quantitatively controlled was also implemented (for micropoint laser ablation) so that by placing a desired site of the blood microvessel on the center of the display of the microscope, cell damage can be produced with a 1-micron light flux. The output energy can be varied to produce from bleeding accompanied by breakthrough bleeding (i.e., accompanied by basal membrane damage) to damage on vascular endothelial cells involving junctional leakage of only a few erythrocytes without the formation of primary platelet aggregates.

Specifically, with this system, different degrees of vascular endothelial cell damage can be produced according to three indexes: (1) the presence of breakthrough bleeding caused by basal membrane damage, (2) the presence of platelet aggregates with the damaged point being the origin, and (3) the presence of erythrocyte leakage to the subendothelial space as recognized in a transmitted light image. Experiments that did not involve these three variations was regarded no damage done with the nitrogen dye laser.

In order to obtain images of the platelets and the Rhodamine B-coated liposomes, a highly sensitive CCD camera (C5810, Hamamatsu Photonics) was equipped in the light path of the microscope while all of the images were saved as 8-bit color digital DVD images. The liposomes were prepared according to a previously described method with an average diameter of 263 nm. After recording the microcirculation image of the control, liposome dispersion (where proportion of liposome in the dispersion is 35% by volume (estimated number of liposomes in the sample being 4.8 x 10¹² /ml)) were infused at 250 µl per rat weighing 100 g for analysis prior to micropoint laser ablation and analysis for 30 minutes following micropoint laser ablation.

### 2. Image analysis method

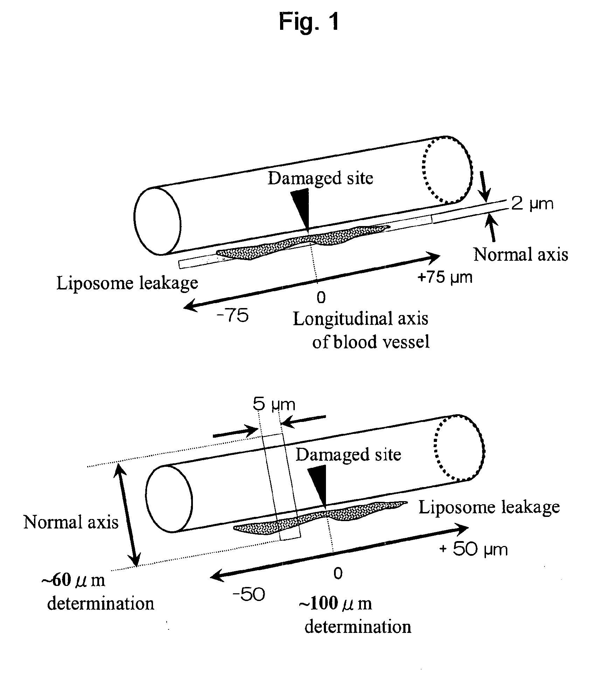

Based on the recorded fluorescent images, semiquantitative analyses of distribution and amount of Rhodamine B-labeled liposomes (hereinafter, also referred to as Rh-lipo) that leaked from the blood vessel were conducted. With respect to the nitrogen laser irradiated site (point of injury, damaged site) on the blood vessel wall, intensity of fluorescence leaked to the subendothelial space was determined along the longitudinal axis or the normal axis of the blood vessel in 8-bit gray level (0-255). Image analysis software (MetaMorph 6.1, Universal Imaging Corporation) was used to determine the gray level along the longitudinal axis of the blood vessel at 2-micron intervals or along the normal at 5-micron intervals as shown in Figure 1. Data of the gray level determination was expressed as average values (standard error) for different arterioles and venulae of four different animals, with the central value being the average of values determined at about 10 sites within each of the unit micron ranges.

### 3. Results

### (1) Liposome behavior in normal microcirculation

With the CFSE-labeling protocol employed in this example, about 80% of the platelets in the circulating blood were stained. These platelets gave an image of a green dashed line on the display. When Rh-lipo was infused, fluorescence momentarily passed across the viewing field and a part thereof slightly stained a suspected junction of the venula endothelial cells and mostly distributed in the blood space. In this case, since the color tones of the CFSE-stained platelets showed no change and gave green color, the administered Rh-lipo was assumed to have a low chance of being incorporated within the platelets. No significant change was observed in the transient adhesion reaction of the platelets to the vascular endothelial cells after Rh-lipo administration. These results prove that administration of liposome has no influence on the behavior of the platelets.

### (2) Liposome behavior associated with micropoint laser ablation

Micropoint laser ablation was performed in arteriole and venula (diameter 20-40 micron) under the conditions described in (1) above with a certain laser energy (laser with an energy pulse of 4-nsec radiated at 5 Hz for about 5 sec., with an output at the laser transmitter exiting port being 300 µJ).

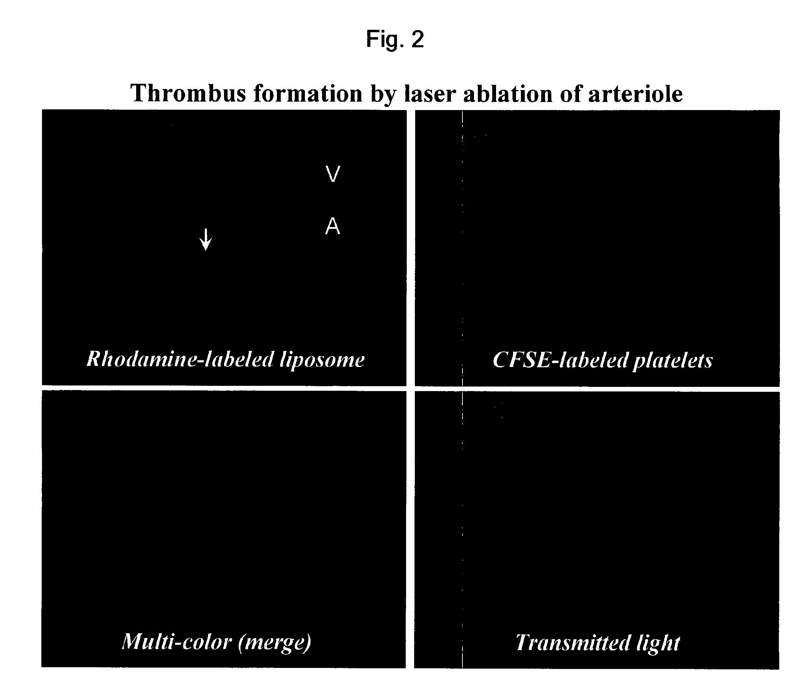

Figure 2 shows the behavior of Rh-lipo after the ablation of arteriole. In the figure, arrows indicate the damaged sites while A and V represent arteriole and venula, respectively. The upper left panel shows behavior of the Rh-lipo; the upper right panel shows behavior of the CFSE-labeled platelets; the lower left panel shows merged image of the behavior of the Rh-lipo and the CFSE-labeled platelets in multi-color; and the lower right panel shows an image obtained with transmitted light. Within a few seconds following ablation, leakage of the liposomes from the blood vessel was confirmed, and at the same time several platelet clots were formed. As can be appreciated from the Rhodamine-labeled fluorescent image in red (Figure 2, upper left panel), liposome leaked from the junction of vascular endothelium in the vicinity of the damage, and, instead of diffusing along the longitudinal axis of the blood vessel, stayed at the boundary (subendothelial space) between the basal membrane and the endothelial cells, showing limited accumulation. These findings prove that the liposome used in this example accumulates specifically on the damaged site of the vascular endothelial cells and fills in the subendothelial space.

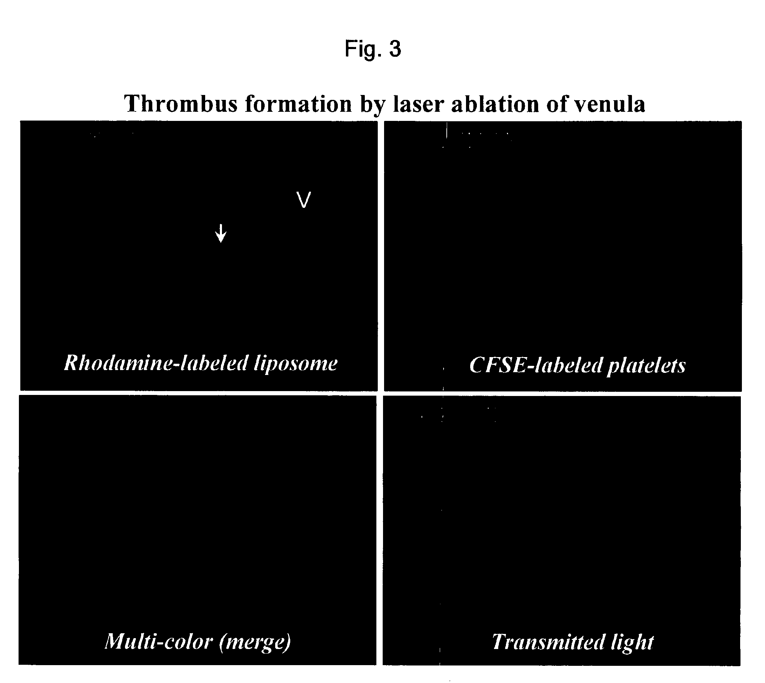

On the other hand, although platelets formed small aggregates based on the damaged point, no overlap with the liposome was recognized and overlap between both fluorescences was small as shown in the merged image (Figure 2, lower left panel). This shows that the negatively charged liposome used in this example has accumulated on the damaged site without depending on an interaction with the platelets. Similar behavior of Rh-lipo was seen when the vascular endothelial cells of the venula were damaged as shown in Figure 3. In venula, however, Rh-lipo was found where platelets adhered to leukocytes rolling in the vicinity of blood vessel wall, or was found partially incorporated into platelet aggregates of liposomes mixing up with leukocytes. In Figure 3, the arrows indicate the damaged sites while V represents venula. The upper left panel shows behavior of the Rh-lipo; the upper right panel shows behavior of CFSE-labeled platelets; the lower left panel shows a merged image of the behavior of Rh-lipo and CFSE-labeled platelets in multi-color; and the lower right panel shows an image obtained with transmitted light.

### (3) Accumulation of Rh-lipo in vascular subendothelial space

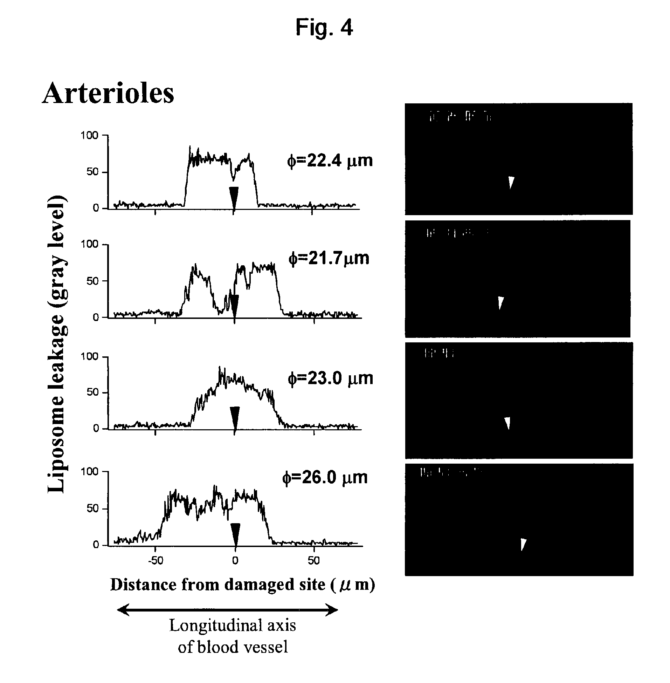

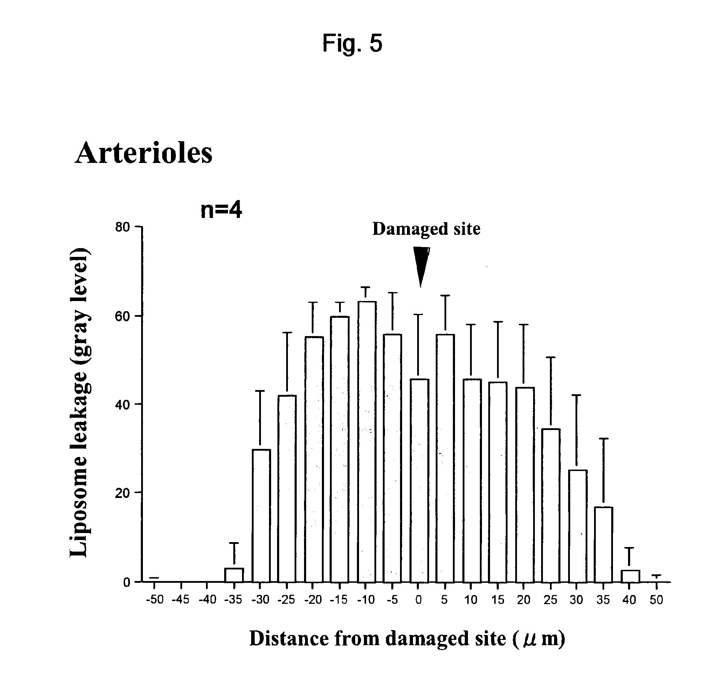

Figure 4 shows the results from analysis of liposome accumulation at the damaged sites of the arterioles. The arrows indicate the damaged sites. The panels on the right in Figure 4 show images of Rh-lipo accumulation at the damaged sites of arterioles with diameters of 22.4, 21.7, 23.0 and 26.0 µm from the top, while the left panels show graphs representing liposome leakages in the arterioles. The analysis took place at least 5 minutes after the ablation when the liposome leakage and the accumulation reach the steady states. The average values of these data are plotted in Figure 5, where liposome accumulation was confirmed to concentrate within about 30 micron from the damaged site. The analysis in the normal direction also showed accumulation localized to the damaged site with little particle mobility to the other side of the damaged site. On the other hand, accumulation in the venulae was within a width of about 40 micron centering around the damaged site. Distribution in the normal direction was also similar to that in the arterioles, although liposome mobility to the blood vessel wall on the other side of the damaged site was occasionally observed.

For both of the blood vessel types, i.e., arterioles and venulae, the liposome used with this example had actions of accumulating on and leaking from the damaged vascular endothelial cell site, and showed accumulation on and filling in the vascular subendothelial space while these actions were independent from the interaction with the platelets.

### EXAMPLE 3: Liposome behavior under inflammation stimulation

The present example aims at revealing liposome behavior upon stimulation of mesenterium with a general inflammation substance instead of mechanical stimulation. Histamine was used as the inflammation stimulating substance. In the same manner as the method of Example 2, male Wistar rats (200 to 250 g) were anesthetized by intramuscular injection of pentobarbital sodium at 50 mg/kg, and a catheter was inserted into a femoral artery. The mesenterium at the ileocecal region was opened extraperitoneally to observe the microcirculatory system with an erecting-type confocal laser microscope. A 60x objective lens was used.

The liposome dispersion prepared according to the method in Example 1 was infused into the femoral vein via the catheter for 250 µl per rat weighing 100 g (250 µl/100g rat, where proportion of liposome in the dispersion was 35% by volume (estimated number of liposomes in the sample being 4.8 x 10¹² /ml)).

Ten minutes after liposome infusion, superfusion (surface perfusion) of histamine (30 µM) began (flow rate: 2ml/min) and liposome behavior was picked up for 10 minutes. The rhodamine-labeled fluorescent images of liposomes in the arterioles (A) and venulae (V) 0 and 2 minutes after the histamine perfusion are shown in the upper left and right panels in Figure 6. The lower panel shows an image obtained with transmitted light 2 minutes after the histamine perfusion.

After 2 minutes of surface perfusion with histamine, large pores of vascular endothelial cells of the venula opened, and leukocytes (WBCs) were confirmed to be rolling and accumulating on the large pores (Figure 6, lower panel). As a result of histamine perfusion, leukocytes that infiltrated to the periphery seemed to liberate cytokines to cause tissue inflammation. The upper right panel in Figure 6 shows that the liposomes accumulate on and fill in the opened large pores. Briefly, liposomes were found to fill in the large pores that are entrances for the leukocytes to enter the tissue, thereby suppressing tissue invasion (accumulation) by the leukocytes.

For inflammation reaction with histamine or the like, the opening of the large pores between the endothelial cells is an important factor for determining the degree of the inflammation reaction. In particular, in the case of acute inflammation caused by histamine, edema is known to be caused by early leakage of body fluid with less protein component from inside the blood vessel to the tissue via the large pores and the subsequent gradual leakage of body fluid containing plasma protein from inside the blood vessel to the tissue. Such inflammation is known to generally occur in the venulae. As can be appreciated from Figure 6, accumulation and filling by the liposome of the invention were observed only in the venulae and were absent in the arterioles.

Thus, this example shows that the liposome of the invention has functions of accumulating on an inflammation site and suppressing the inflammation.

### EXAMPLE 4: Assessment of plasma leakage in each of the mouse tissues

In this example, Evans Blue tracer was used to test and assess plasma leakage in blood microvessels of various tissues (brain, liver, kidney and lung) of a mouse (C57BL/6).

### (1) Preparation of liposome

Liposomes were prepared in the same manner as Example 1 to obtain a liposome dispersion with an average particle size of 247± 129 nm, a volume ratio of 35% in the dispersion and particle concentration of about 4.5 x 10⁶/µl.

### (2) Test method

A mouse (C57BL/6) was anesthetized with urethane (600 mg/kg)/α-chloralose (60 mg/kg), and cannula was inserted into the vein of the femoral region following tracheal intubation (spontaneous ventilation).

The liposome dispersion was infused from the vein of the femoral region at 5 µl/g mouse weight. Next, in order to prepare a brain ischemia model, bilateral common carotid artery (BCCA) was occluded for 1 hour before being subjected to reperfusion.

Evans Blue was infused from the vein of the femoral region. The stock solution of Evans Blue dye was 3% w/v (in PBS) that had been filtrated through a filter and stored at 4°C. The stock solution was 4-fold diluted in PBS (1% solution) and the amount infused was 5 µl/g mouse weight (50mg/kg).

The above-described perfusion (ischemia perfusion) was continued for 4 hours, followed by heart perfusion with 100mM citrate buffer (pH3.5) at 37°C. When the mouse tissue was used histologically, it was perfused with 1% paraformaldehyde dissolved in 50mM citric acid. By fixing pH to a low value, binding between Evans Blue and albumin was retained. The ventral part of the mouse was pinned on a corkboard, and chest cavity was opened to expose the heart. 21G butterfly needle was inserted into the left ventricle (just right to the apical end), followed by perfusion. Small cut was made in the left atrium. It is very important to completely wash away any blood and Evans Blue from the vessel as described above.

Each of the mouse tissues (brain, liver, kidney and lung) was washed briefly with a citrate buffer and carefully wiped with paper towel. After weighing each of the tissues, each of them was placed in a 4ml glass vial together with 0.5ml of formamide.

Each glass vial was agitated at 55°C overnight, and Evans Blue was extracted. On the following morning, 100 µl each of the formamide-extracted sample was transferred to a cuvette together with the standard Evans Blue solution to determine the amount of Evans Blue in each sample with a spectrophotometer (OD₆₂₀ to OD₇₅₀).

From the determined OD value, concentration of Evans Blue (ng/mg) was determined by a conversion using a standard curve. Evans Blue was added to 1ml of formamide and agitated at 55°C overnight before use. The converted value is expressed as an amount of Evans Blue (ng) to the weight of each mouse tissue (mg).

A control that omitted the bilateral common carotid artery (BCCA) occlusion and the subsequent perfusion (ischemia perfusion) in the above-described test method (Sham) as well as a control that omitted the liposome infusion (Vehicle) in the above-described test method were also examined.

### (3) Test results and assessment

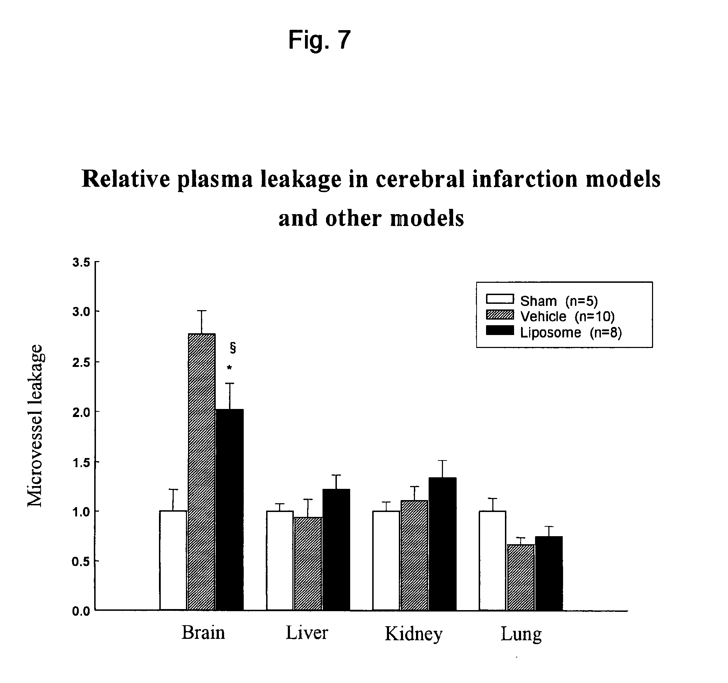

The results are shown in Figures 7 to 9.

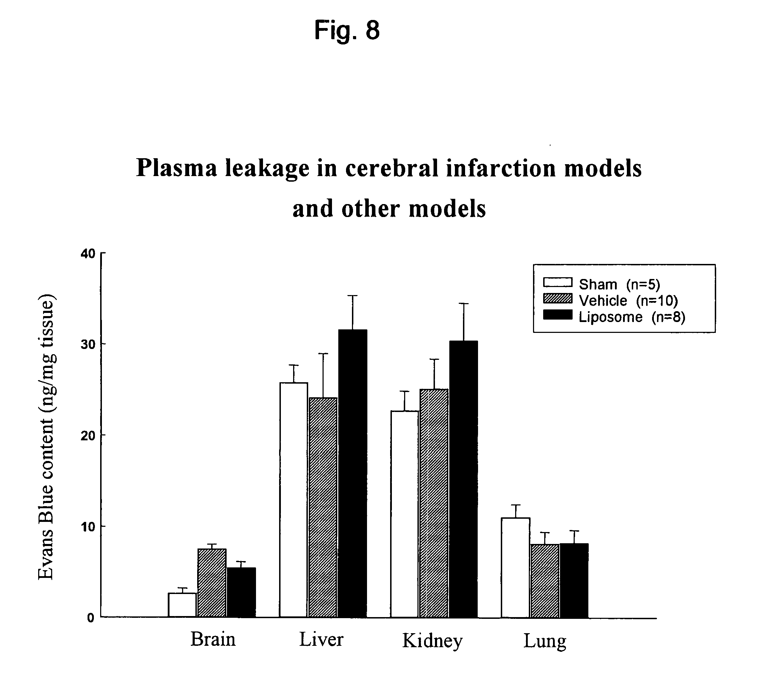

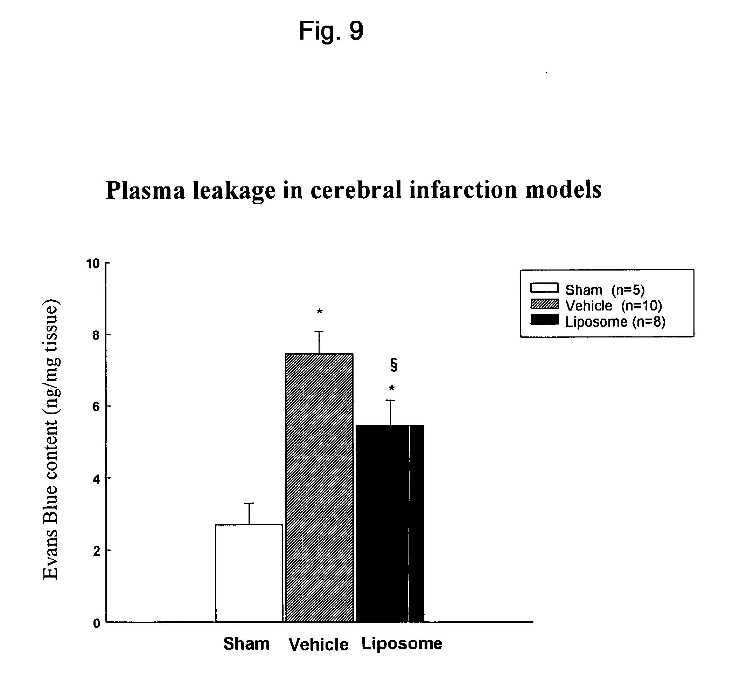

Figure 7 shows relative Evans Blue concentrations (ng/mg) in various tissues (brain, liver, kidney and lung) with the vehicle and the present example where the Evans Blue concentration in the sham control is taken as 1.0. Figure 8 shows the Evans Blue concentration (ng/mg) in each tissue in an absolute value and Figure 9 is an enlarged view of the brain.

From these results, among the various tissues, plasma leakage in brain with the vehicle control was particularly significant as compared to that with the sham control while plasma leakage with the present example was found reduced effectively. In the brain, kidney and lung without the ischemia operation, no increase in the Evans Blue leakage was recognized. Thus, the liposome of the invention was found to effectively suppress or inhibit an inflammation-induced damage such as brain edema in the blood microvessels in the brain tissue if such event occurs or if such event is likely to occur.

### INDUSTRIAL APPLICABILITY

The present invention provides a carrier with a non-cationic surface, which can attach to and accumulate on a damaged site of a tissue and which can specifically fill in the damaged site, and a pharmaceutical composition comprising such carrier. The pharmaceutical composition of the invention may be used as a drug for controlling platelet functions such as hemostasis action and antithrombotic action. In addition, the pharmaceutical composition of the invention may be used as a drug delivery system (DDS) for a damaged blood vessel site.

Furthermore, the present invention allows observation of carrier behavior within a tissue in multi-color and in real time by using a high-speed confocal microscope. A method of the invention allows direct observation of dynamics of a carrier within a blood vessel, and thus can be used for research and development of a novel drug carrier or a highly selective drug carrier.

## Claims

1. A carrier with a non-cationic surface, which can accumulate on a damaged site of a tissue.

2. A carrier according to claim 1, wherein the surface is a membrane.

3. A carrier according to either one of claims 1 and 2, wherein the tissue is a vessel.

4. A carrier according to claim 3, which can diffuse outside the vessel.

5. A carrier according to either one of claims 3 and 4, wherein the vessel is a blood vessel.

6. A carrier according to claim 1, wherein the damage reaches an endothelial cell.

7. A carrier according to claim 1, wherein the damage comprises those that result from laser, inflammation, ischemic disorder, ischemia-reperfusion damage, bacterial toxin, oxidative stress, tumor or thrombus formation, or bleeding.

8. A carrier according to claim 7, wherein the inflammation is brain edema.

9. A carrier according to claim 7, wherein the ischemic disorder is cerebral ischemic disorder.

10. A carrier according to claim 7, wherein the ischemia-reperfusion damage is ischemia-reperfusion-induced organ damage.

11. A drug transporter comprising the carrier of any one of claims 1 to 10.

12. A pharmaceutical composition comprising the drug transporter of claim 11 incorporating or carrying a drug.

13. A pharmaceutical composition according to claim 12, which functions as a drug for controlling a platelet function.

14. A pharmaceutical composition according to claim 13, wherein the platelet function to be controlled comprises hemostasis, antithrombotic formation, thrombolysis or antiatherogenic action.

15. A pharmaceutical composition according to claim 12, wherein the drug is at least one selected from a group consisting of substances that are activated by light, change in temperature, change in pH, ultrasound, uptake of an inflammation-mediating cell or enzyme degradation; hemostatic agents; antithrombotic agents; thrombolytic agents; antitumor agents; and antiatherogenic agents.

16. A pharmaceutical composition according to claim 15, wherein the inflammation-mediating cell is a lymphocyte, a leukocyte, a macrophage or a platelet.

17. A drug delivery method comprising allowing the pharmaceutical composition according to any one of claims 12 to 16 to accumulate on a damaged site of a tissue.

18. A drug control method comprising allowing the pharmaceutical composition according to any one of claims 12 to 16 to accumulate on a damaged site of a tissue and allowing the drug to act on the damaged site.

19. A method according to claim 18, wherein the action of the drug is controlled by accumulation of the carrier, diffusion of the carrier or activation of the carrier.

20. A method according to any one of claims 17 to 19, wherein the tissue is a vessel.

21. A method according to claim 20, wherein the vessel is a blood vessel.

22. A method for assessing a function of a carrier comprising observing the behavior of the carrier within a tissue under a high-speed confocal widefield microscope.

23. A method according to claim 22, wherein the observation of the carrier behavior within a tissue is performed in multi-color and in real time.

24. A method according to claim 22, wherein the carrier has a non-cationic surface.

25. A method according to claim 24, wherein the surface is a membrane.

26. A method according to claim 22, wherein the tissue is a vessel.

27. A method according to claim 26, wherein the vessel is a blood vessel.
